Europäisches Patentamt

(19) European Patent Office

Office européen des brevets

(11) Publication number : **0 321 249 B1**

(12) **EUROPEAN PATENT SPECIFICATION**

(45) Date of publication of patent specification :
25.01.95 Bulletin 95/04

(51) Int. Cl.[6] : **G01N 33/569,** G01N 33/577, C12P 21/00, G01N 33/68

(21) Application number : 88311887.9

(22) Date of filing : 15.12.88

(54) **Agent for and method of diagnosis of varicella zoster virus infection.**

(30) Priority : 15.12.87 JP 315254/87
01.11.88 JP 274602/88

(43) Date of publication of application :
21.06.89 Bulletin 89/25

(45) Publication of the grant of the patent :
25.01.95 Bulletin 95/04

(84) Designated Contracting States :
DE FR GB

(56) References cited :
EP-A- 0 148 644
EP-A- 0 198 086
WO-A-86/02364
JOURNAL OF CLINICAL MICROBIOLOGY, vol. 18, no. 2, August 1983, American Society for Microbiology, Washington, DC (US); K.S. KIM et al., pp. 331-343
VIROLOGY, vol. 129, 1983, T. OKUNO, pp. 357-368

(73) Proprietor : TOA NENRYO KOGYO KABUSHIKI KAISHA
1-1 Hitotsubashi 1-chome
Chiyoda-ku Tokyo (JP)

(72) Inventor : Sugimoto, Masanobu, c/o Toa Nenryo Kogyo K.K.
Corporate Res. & Dev. Lab.,
1-3-1 Nishitsurugaoka
Oimachi, Iruma-gun, Saitama (JP)
Inventor : Nishimaki, Junichi, c/o Toa Nenryo Kogyo K.K.
Corporate Res. & Dev. Lab.,
1-3-1 Nishitsurugaoka
Oimachi, Iruma-gun, Saitama (JP)
Inventor : Oomori, Ikuyo, c/o Toa Nenryo Kogyo K.K.
Corporate Res. & Dev. Lab.,
1-3-1 Nishitsurugaoka
Oimachi, Iruma-gun, Saitama (JP)

(74) Representative : Baverstock, Michael George Douglas et al
BOULT, WADE & TENNANT
27 Furnival Street
London, EC4A 1PQ (GB)

EP 0 321 249 B1

## Description

The present invention relates to an agent for and method of diagnosing a varicella zoster virus infection.

The varicella zoster virus disease is an acute virus disease accompanied by a sudden fever and eruptions. The virus usually infects infants, and for three to four days, produces varicella from crusts which finally become granular crusts, and the disease is then cured. If, however, the virus remains latent in ganglion of the posterior root of the spinal nerve, a recurrence can occur many years later to again produce zoster.

Currently, antibodies to the human herpes simplex virus belonging to the herpes virus family to which varicella zoster also belongs are used as the diagnostic agent, but although these antibodies react with the human herpes simplex virus, they do not react with the varicella zoster virus. Therefore, the development of diagnostic agent by which the varicella zoster virus can be detected is urgently desired.

An antibody against glycoprotein GP of varicella zoster virus is known from Virology, 129, 357-368, 1983. Moreover, an antibody against a nucleocapsid NP of the varicella zoster virus is known. But, a single use of the antibody does not provide a sufficient sensitivity or a clear result.

Therefore, the object of the present invention is to provide a diagnostic agent and diagnostic method directed to the varicella zoster virus disease, using a combination of two kinds of antibodies, which provide a highly sensitive detection and a clear diagnostic result.

The method of diagnosing a varicell a zoster virus infection disease, in accordance with the invention uses a monoclonal antibody to glycoprotein GP of the varicella zoster virus, said monoclonal antibody being secreted by the hybridoma having the deposit number FERM BP-2123, and a monoclonal antibody to nucleocapsid NP of the varicella zoster virus, said monoclonal antibody being secreted by the hybridoma having the deposit number FERM BP-2124, which are placed in contact with a sample comprising cells, and a specific binding of both antibodies with cells infected with the virus is detected.

Moreover, the present invention provides a diagnostic agent and a diagnostic kit for varicella zoster virus infection disease each comprising said monoclonal antibodies described above.

In particular a method of diagnosing varicella zoster virus infection disease in accordance with the invention comprises placing a monoclonal antibody to glycoprotein GP of varicella zoster virus and a monoclonal antibody to nucleocapsid NP of varicella zoster virus in contact with a sample comprising cells, and detecting a specific binding of both said antibodies with cells infected with said virus, characterized in that the monoclonal antibody to the glycoprotein GP is a monoclonal antibody produced by a hybridoma having the deposit No FERM BP-2123, which monoclonal antibody strongly binds to the cytoplasm of a cell infected with the varicella zoster virus, and the monoclonal antibody to the nucleocapsid NP is a monoclonal antibody produced by a hybridoma having the deposit No FERM BP-2124, which monoclonal antibody strongly binds to the nucleus of a cell infected with the varicella zoster virus wherein both of the antibodies are labelled with fluorescein isothiocyanate.

The present invention also provides a diagnostic agent and a diagnostic kit comprising the two monoclonal antibodies described above which are produced by hybridomas FERM BP-2123 and FERM BP-2124 respectively.

The invention will now be described by way of the preferred embodiments and with reference to the accompanying drawings in which:-

Figure 1 is a fluorescence micrograph showing cells stained with monoclonal antibodies optimally labelled with fluorescein isothiocyanate; and,

Fig. 2 is a fluorescence micrograph showing cells stained with monoclonal antibodies excessively labelled with fluorescein isothiocyanate.

It has, after testing various monoclonal antibodies, been found that, as a monoclonal antibody to the glycoprotein GP, a monoclonal antibody produced by hybridoma No. 8 (FERM BP-2123) is most suitable and as a monoclonal antibody to the nucleocapsid NP, a monoclonal antibody produced by hybridoma No. 17 (FERM BP-2124), is most suitable. Of these monoclonal antibodies, the former strongly binds to cytoplasm of a cell infected with the varicella zoster virus (target cell), and the latter strongly binds to a nucleus of a target cell. Therefore, a combination of the above-mentioned particular monoclonal antibodies is most preferable.

According to the present invention, a combination of the above-mentioned two monoclonal antibodies is used. Although the ratio of the monoclonal antibodies is not critical, a ratio of about 1:1 is preferable.

For a diagnosis, a sample is taken from a patient by rubbing a lesion, to isolate cells of a lesion from basal raxer, with a cotton swab, and the cells are fixed with methanol or acetone, preferably acetone, on a slide glass. The fixed cells are then placed in contact with the above-mentioned monoclonal antibodies to allow a specific binding of the monoclonal antibodies to target cells. In this case, the monoclonal antibody to the glycoprotein GP binds to cytoplasm of the target cell, and the monoclonal antibody to the nucleocapsid NP binds to a nucleus of a target cell.

Methods fo detecting antibodies which are bound to the target cell include a direct method wherein the above-mentioned antibodies labelled with a label are used. An indirect method is also known wherein a labelled second antibody against the above-mentioned antibodies would be used. In the present invention however the direct method is used wherein the above-mentioned two monoclonal antibodies labelled with a same label are used. For labelling, the fluorescein isothiocyanate method is used. The fluorescein isothiocyanate method provides an easy detection of stained cells under a fluorescence microscope.

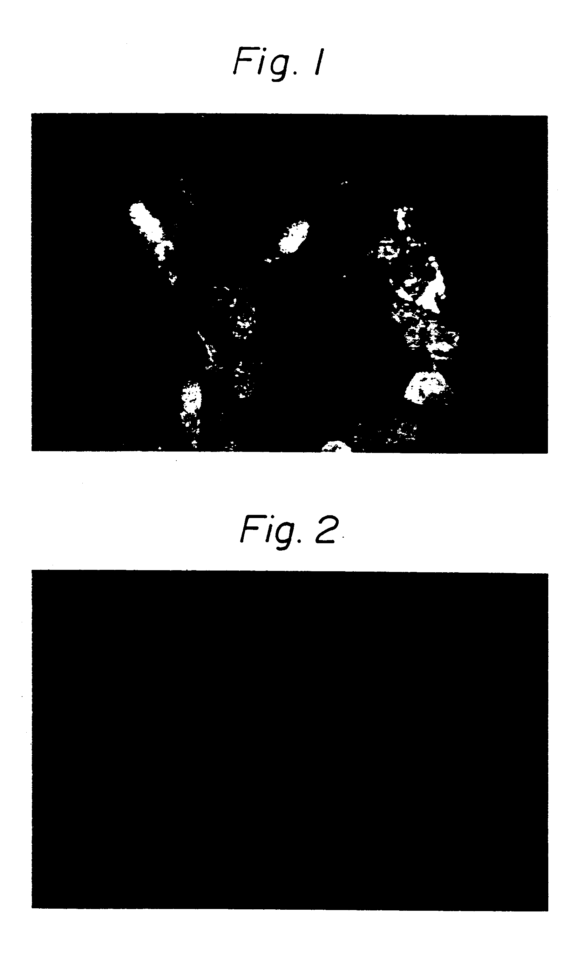

According to the present invention, the mole ratio of fluorescein isothiocyanate and the antibody protein in a labeled antibody is preferably about 2.5 to 3.5. Where the ratio of fluorescein isothiocyanate is too low, the target cell is insufficiently stained, and detection of stained cells is difficult, but where the ratio of fluorescein isothiocyanate is too high, the monoclonal antibody excessively denaturated, and thus it becomes difficult to detect the target cells. Figure 1 represents a fluorescence micrograph showing cells stained with the monoclonal antibodies labeled in an adequate ratio of the label and the antibody; and Fig. 2 represents a fluorescein micrograph showing cells stained with the excessively labeled monoclonal antibodies.

To prepare a monoclonal antibody labeled with fluorescein isothiocyanate in a preferable ratio, the antibody protein and fluorescein isothiocyanate should be allowed to react at a weight ratio of about 160:1 to 200:1. In a most preferable procedure, an antibody protein solution at a concentration of about 20 to 25 mg/ml, and a fluorescein isothiocyanate solution at a concentration of about 1 mg/ml are prepared, and the protein solution and the fluorescein isothiocyanate solution are mixed in a volume ratio of about 8:1, to allow a reaction. As a reaction medium, a carbonate buffer (pH 9.5) is preferably used, although other buffers can be used. The reaction is preferably carried out at a temperature of between 2°C and 8°C for 10 to 20 hours.

The labeled antibody thus prepared in a basic carbonate buffer is then purified on a column equilibrated with a neutral phosphate buffer to obtain a solution of the labeled antibody in the neutral phosphate buffer. Preferably, to the labeled antibody Evans' Blue in an amount of 0.001% relative to the medium (W/V) is added. In this way, cells or parts of a cell to which the fluorescein isothiocyanate-labeled antibodies are not bound, are stained with Evans' Blue, i.e., are colored red, thereby parts of a cell stained by fluorescein isothiocyanate via the antibody can be clearly detected.

EXAMPLES

The present invention will now be further illustrated by, but is in no way limited to, the following Examples.

Example 1. Preparation of hybridoma

Human embryo lung cells (HEL) were infected with Kawaguchi strain of varicella zoster virus, and after confirming a development of the cytopathic effect, a cell layer was washed with a phosphate buffer and peeled off. The cells were sonicated and the sonicate was stored at -80°C, to be used as an antigen. Mice were intraperitoneally injected with the sonicate in an amount of 0.5 ml/mouse and 0.5 ml/mouse of Freund's complete adjuvant, and after one month, the same amount of the sonicate was intraperitoneally injected as a boost without an adjuvant. Three days after the boost, the spleens of the mice were separated and subjected to cell fusion.

The spleen cells were washed with MEM medium, and treated with 155mM ammonium chloride solution to lyse erythocytes. Remaining lymphocytes were further washed with the medium, and mixed with myeloma SP2/10-Ag14 cells at a ratio of 10 : 1, and the mixture was centrifuged. Polyethylene glycol No. 4000 was added to the cell pellet to accelerate the cell fusion, and fused cells were selected in a medium containing hypoxanthine, aminopterin and thymidine (HAT medium).

A supernatant from each colony grown in the HAT medium was tested by an indirect immunofluorescence technique. Namely, human embryo lung cells were infected with the varicella zoster virus, and after confirming the cytopathic effect, the above-mentioned supernatant from the hybridoma was overlayed to the cell layer. The cells were incubated at 37°C for 60 minutes and washed with a phosphate buffer. Next, anti-mouse IgG-fluorescein isothiocyanate was overlayed to the washed cells, the cells were incubated at 37°C for 30 minutes, and then washed with a phosphate buffer. The resultant cells were observed by a fluorescence microscopy, and specifically stained cells with an apple green were determined. Colonies corresponding to the specifically stained human embryo lung cells were selected as hybridoma producing a desired monoclonal antibody to the varicella zoster virus, and as a result, hybridoma No. 8, which produces a monoclonal antibody recognizing glycoprotein GP of the varicella zoster virus, and hybridoma No. 17 which produces a monoclonal antibody recognizing nucleocapsid NP of the varicella zoster virus, were obtained.

Note, the starting cells used in the above procedure are easily available in the art.

The above-mentioned hybridoma No. 8 was designated as VZV-MAb-cl 8, and deposited with the Fermentation Research Institute Agency of Industrial Science and Technology (FRI), 1-3, Higashi 1-chome, Tsukuba-shi, Ibaraki-ken, Japan, as FERM BP-2123, on October 25, 1988, as an international deposition under the Budapest Treaty. The hybridoma No. 17 was designated as VZV-MAb-cl 17, and deposited with the FRI as FERM BP-2124, on October 25, 1988.

Example 2. Preparation of monoclonal antibodies

Each of the above-mentioned hybridoma was cultured in a DMEM-NCTC109 medium having the following composition:

| | |
|---|---|
| DMEM[1] | 1000 ml |
| NCTC 109 | 150 ml |
| NEAA (*100) | 15 ml |
| 100 mM Pyruvic acid | 7 ml |
| 3% Glutamine | 15 ml |
| Solution I[2] | 15 ml |
| Gentamycin (10 mg/ml) | 1.5 ml |

(1) DMEM

| | |
|---|---|
| DMEM Powder | 1 pack |
| $NaHCO_3$ | 2.0 g |
| L-Glutamine | 0.584 g |
| Sodium pyruvate | 0.110 g |
| Penicillin G | $1 \times 10^5$ IU |
| Streptomycin | 0.1 g unit |
| Distillated water | to a total of 1000 ml |

(2) Solution I

| | |
|---|---|
| Oxaloacetic acid | 100 mM |
| Bovine insulin | 20 IU/ml |

Pristane was intraperitoneally injected to BALB/C mice in an amount of 0.5 ml/mouse, and after three weeks, $1 \times 10^7$ of the above-cultured hybridoma cells were intraperitoneally injected to the mouse. The above-mentioned medium was supplemented with 15% fetal calf serum. One to two weeks after, ascites was obtained and was centrifuged to eliminate cells, and the supernatant was subjected to salting out with ammonium sulfate. A precipitated fraction obtained at between a 20% and 40% saturation was dissolved in a 0.05 M carbonate buffer (pH 9.5).

Example 3. Preparation of labeled antibody

A solution of the antibody purified by ammonium sulfate precipitation as described above in carbonate buffer was adjusted to a concentration of at least 35 mg/ml, and 2.5 ml of the solution was applied to a PD-10 column Sephadex G-25, (Registered Trade Mark) equilibrated with a carbonate buffer. Then 3.5 ml of the carbonate buffer was flowed through the column to recover 3.5 ml of elute containing the antibody, and the solution was then adjusted to a protein concentration of about 20 to 25 mg/ml with a carbonate buffer. In this case, the protein concentration was defined as follows:

Protein concentration = $A_{280}/1.4$.

On the other hand, 1 mg of powdery fluorescein isothiocyanate was dissolved in 1 ml of a carbonate buffer (0.5 M $Na_2CO_3$-$NaHCO_3$; pH 9.5) to obtain 1 μg/μl of a fluorescein isothiocyanate solution.

Next, the fluorescein isothiocyanate solution and the antibody solution were mixed at a ratio of 125 μl (0.125 mg) : 1000 μl (20 to 30 mg), and the mixture was incubated at 4°C for about 15 hours, in the dark, while stirring to allow the reaction of the fluorescein isothiocyanate and the antibody protein.

The reaction mixture was applied to a PD-10 column equilibrated with a 0.01 M phosphate buffer (pH 7.2; containing 0.1 M NaCl and 0.02% $NaN_3$), and an elution was carried out using the same phosphate buffer to obtain a yellow-color fraction containing an antibody labeled with fluorescein isothiocyanate. During this procedure, free fluorescein isothiocyanate remained in the column. Next, the antibody-containing fraction was applied to an ion exchange column QAE Zetaprep, and 50 ml of a 0.01 M phosphate buffer (pH 7.2; containing 0.1 M NaCl) was passed through the column to elute the remaining free fluorescein isothiocyanate and nonreacted antibody. Next, 35 to 40 ml of a 0.01 M phosphate buffer (pH 7.2; containing 0.5 M NaCl) was passed through the column to recover the optimally labeled antibody. The recovered fraction was diluted 10-fold with a phosphate buffer (pH 7.2; containing 0.1 M NaCl and 0.02% $NaN_3$), and the absorption spectrum of the diluted solution was measured at 530 to 250 nm. As a result, two peaks at 495 nm and 280 nm, which represent fluorescein isothiocyanate (F) and protein (P), respectively, were observed. On the basis of the values of the peaks, a ratio of fluorescein isothiocyanate and antibody protein, i.e., a F/P ratio, was calculated as follows:

$$\text{Protein concentration} = A_{280} \times 0.75 - A_{495} \times 0.227$$
$$\text{F/P ratio} = A_{495} \times 2.34/\text{protein concentration}$$

As a result, it was confirmed that the F/P ratio was 2.5 to 3.5.

About 0.001% (final concentration) Evans' Blue was added to the antibody solution as a dye for counterstaining.

In the above-mentioned procedure, two monoclonal antibody solutions from hybridoma No. 8 and No. 17 were prepared, and these solutions were mixed at a volume ratio of 1 : 1 to obtain a final diagnostic solution.

## Example 4. Detection of infected cells

A cell sample was taken from a lesion base laxer of a patient infected with the varicella zoster virus, using a cotton swab, and the cells were smeared on three slide glasses (A, B, and C), at two sites on each slide glass, and dried in air. The cells were fixed with acetone and the acetone was then evaporated. A drop of a solution of the labeled monoclonal antibody produced by hybridoma No. 8 was added to each spot of cells on the slide glass A; a drop of a solution of the labeled monoclonal antibody produced by hybridoma No. 17 was added to each spot of cells on the slide glass B; and a drop of the mixed solution prepared in Example 3 containing both the labeled monoclonal antibodies produced by hybridoma No. 8 and No. 17 was added to each spot on the slide glass C. The slide glasses were incubated at 37°C for 30 minutes, washed with a washing solution PBS(-) to eliminate excess agents, and dried. A drop of an inclusion fluid was added to each spot of cells, a cover slit was placed over the spot, and the cells were observed under a fluorescence microscope.

As a result, cells treated with the labeled monoclonal antibody derived from hybridoma No. 8 were weakly stained at the cytoplasm thereof, an cells treated with the labeled monoclonal antibody derived from hybridoma No. 17 were weakly stained at the nucleus thereof. In both cases, the stain was not clear. Conversely, cells treated with a mixture of both the labeled monoclonal antibodies derived from hybridoma No. 8 and hybridoma No. 17 were stained at both the cytoplasm and nucleus thereof, and clearly stained overall.

Note, although cells not infected with the varicella zoster virus were treated by the same procedure as described above, the cells were not stained by any of the labeled monoclonal antibodies and mixtures thereof.

Figure 1 represents a fluorescence micrograph of cells treated with the mixture of the labeled monoclonal antibodies.

## Example 5. Diagnostic kit

(1) Two ml each of the labeled monoclonal antibody solutions prepared in Example 3 were filled in different vials to prepare a diagnostic kit. This kit can be stored at a temperature lower than 5°C. The solutions are mixed at a volume ratio of 1 : 1 prior to use.

(2) Two ml each of the labeled monoclonal antibody solutions prepared in Example 3 were filled in different vials, and lyophilized to prepare a diagnostic kit. This kit can be stored at a temperature lower than 5°C. Immediately before use, 2 ml each of distilled water is added to the ampoules and the lyophilizates dissolved. The solutions are mixed at a volume ratio of 1 : 1.

(3) Two ml of the mixture of the labeled monoclonal antibody solutions prepared in Example 3 was filled in vial. This kit can be stored at a temperature lower than 5°C.

(4) Two ml of the mixture of the labeled monoclonal antibody solutions prepared in Example 3 was filled in vial, and lyophilized. This kit can be stored at a temperature lower than 5°C. The lyophilizate is dissolved in 2 ml of distilled water immediately before use.

Example 6

223 patients, previously diagnosed as infected with the varicella zoster virus by a clinical diagnosis, were diagnosed by the method described in Example 4, and as a result, 215 patients were found to test positive for the varicella zoster virus infection, i.e., the ratio of positive indication was more than 96%.

**Claims**

1. A method of diagnosing varicella zoster virus infection disease, comprising placing a monoclonal antibody to glycoprotein GP of varicella zoster virus and a monoclonal antibody to nucleocapsid NP of varicella zoster virus in contact with a sample comprising cells, and detecting a specific binding of both said antibodies with cells infected with said virus, characterized in that the monoclonal antibody to the glycoprotein GP is a monoclonal antibody produced by a hybridoma having the deposit No FERM BP-2123, which monoclonal antibody strongly binds to the cytoplasm of a cell infected with the varicella zoster virus, and the monoclonal antibody to the nucleocapsid NP is a monoclonal antibody produced by a hybridoma having the deposit No FERM BP-2124 which monoclonal antibody strongly binds to the nucleus of a cell infected with the varicella zoster virus wherein both of the antibodies are labelled with fluorescein isothiocyanate.

2. A method as claimed in claim 1, wherein the mole ratio of fluorescein isothiocyanate and monoclonal antibody is between 2.5 and 3.5.

3. A method as claimed in claim 1 or claim 2 wherein said labelled monoclonal antibodies are used in combination with Evans' Blue.

4. A diagnostic agent for varicella zoster virus infection disease comprising, a monoclonal antibody to glycoprotein GP of varicella zoster virus and a monoclonal antibody to nucleocapsid NP of varicella zoster virus, characterized in that the monoclonal antibody to the glycoprotein GP is a monoclonal antibody produced by a hybridoma having the deposit No FERM BP-2123, which monoclonal antibody strongly binds to the cytoplasm of a cell infected with the varicella zoster virus, and the monoclonal antibody to the nucleocapsid NP is a monoclonal antibody produced by a hybridoma having the deposit No FERM BP-2124, which monoclonal antibody strongly binds to the nucleus of a cell infected with the varicella zoster virus wherein both of the antibodies are labelled with fluorescein isothiocyanate.

5. A diagnostic agent as claimed in claim 4 wherein both monoclonal antibodies are mixed together.

6. A diagnostic agent as claimed in claim 4 or claim 5 wherein the mole ratio of fluorescein isothiocyanate and monoclonal antibody is between 2.5 and 3.5.

7. A diagnostic agent as claimed in any one of claims 4, 5 or 6 wherein Evans' Blue is added to the labelled monoclonal antibodies.

8. A diagnostic kit for varicella zoster virus infection disease comprising a monoclonal antibody to glycoprotein GP of varicella zoster virus and a monoclonal antibody to nucleocapsid NP of varicella zoster virus, characterized in that the monoclonal antibody to the glycoprotein GP is a monoclonal antibody produced by a hybridoma having the deposit No FERM BP-2123, which monoclonal antibody strongly binds to the cytoplasm of a cell infected with the varicella zoster virus, and the monoclonal antibody to the nucleocapsid NP is a monoclonal antibody produced by a hybridoma having the deposit No FERM BP-2124, which monoclonal antibody strongly binds to the nucleus of a cell infected with the varicella zoster virus wherein both of the antibodies are labelled with fluorescein isothiocyanate.

9. A diagnostic kit as claimed in claim 8, wherein the mole ratio of fluorescein isothiocyanate and monoclonal antibody is between 2.5 and 3.5.

10. A diagnostic kit as claimed in claim 8 or claim 9 wherein Evans' Blue is added to the labelled monoclonal antibodies.

11. A diagnostic kit as claimed in any one of claims 8 to 10 comprising:-
    (a) a solution containing the two monoclonal antibodies or

(b) a lyophilizate containing the two monoclonal antibodies or
(c) separate solutions of each of the monoclonal antibodies or
(d) separate lyophilizates of each of the monoclonal antibodies.

**Patentansprüche**

1.  Verfahren zum Diagnostizieren einer Varicella zoster-Virusinfektionserkrankung, welche umfaßt:
    In-Kontakt-Bringen eines monoklonalen Antikörpers gegen Glycoprotein GP eines Varicella zoster-Virus und eines monoklonalen Antikörpers gegen das Nukleoclapsid NP des Varicella zoster-Virus mit einer Probe, welche Zellen umfaßt, und Bestimmen eines spezifischen Bindens beider Antikörper mit Zellen, die mit dem Virus infiziert sind,
    dadurch gekennzeichnet, daß
    der monoklonale Antikörper gegen das Glycoprotein GP ein monoklonaler Antikörper ist, welcher durch ein Hybridom hergestellt wird, mit der Hinterlegungsnummer FERM BP-2123, wobei der monoklonale Antikörper stark an das Cytoplasma einer Zelle bindet, welche mit dem Varicella zoster-Virus infiziert ist, und der monoklonale Antikörper gegen das Nukleocapsid NP ein monoklonaler Antikörper ist, welcher hergestellt wird durch ein Hybridom mit der Hinterlegungsnummer FERM BP-2124, wobei der monoklonale Antikörper stark an den Kern einer Zelle bindet, welche mit dem Varicella zoster-Virus infiziert ist, wobei beide Antikörper mit Fluorescein-isothiocyanat markiert sind.

2.  Verfahren nach Anspruch 1, wobei das Molverhältnis von Fluoresceinisothiocyanat und monoklonalem Antikörper zwischen 2,5 und 3,5 liegt.

3.  Verfahren nach Anspruch 1 oder 2, wobei die markierten monoklonalen Antikörper in Kombination mit Evans' Blue verwendet werden.

4.  Diagnosemittel für eine Varicella zoster-Virusinfektionserkrankung mit:
    einem monoklonalen Antikörper gegen Glycoproteine GP des Varicella zoster-Virus und einem monoklonalen Antikörper gegen Nukleocapsid NP des Varicella zoster-Virus,
    dadurch gekennzeichnet, daß
    der monoklonale Antikörper gegen das Glycoprotein GP ein monoklonaler Antikörper ist, welcher durch ein Hybridom hergestellt wird, mit der Hinterlegungsnummer FERM BP-2123, wobei der monoklonale Antikörper stark an das Cytoplasma einer Zelle bindet, die mit Varicella zoster-Virus infiziert ist, und der monoklonale Antikörper gegen das Nucleocapsid NP ein monoklonaler Antikörper ist, der durch ein Hybridom mit der Hinterlegungsnummer FERM BP-2124, wobei der monoklonale Antikörper stark an den Kern der Zelle, die mit dem Varicella zoster-Virus infiziert ist, bindet, wobei beide Antikörper mit Fluoresceinisothiocyanat markiert sind.

5.  Diagnosemittel nach Anspruch 4, wobei beide monoklonalen Antikörper miteinander gemischt sind.

6.  Diagnosemittel nach Anspruch 4 oder 5, wobei das Molverhältnis von Fluoresceinisothiocyanat und monoklonalem Antikörper zwischen 2,5 und 3,5 liegt.

7.  Diagnosemittel nach irgendeinem der Ansprüche 4, 5 oder 6, wobei Evans' Blue zu den markierten monoklonalen Antikörpern zugegeben wird.

8.  Diagnosekit für eine Varicella zoster-Virusinfektionserkrankung mit:
    einem monoklonalen Antikörper gegen Glycoprotein GP des Varicella zoster-Virus und einem monoklonalen Antikörper gegen Nukleocapsid NP des Varicella zoster-Virus,
    dadurch gekennzeichnet, daß
    der monoklonale Antikörper gegen das Glycoprotein GP ein monoklonaler Antikörper ist, der durch ein Hybridom mit der Hinterlegungsnummer FERM BP-2123 hergestellt wird, wobei der monoklonale Antikörper stark an das Cytoplasma einer Zelle bindet, die mit dem Varicella zoster-Virus infiziert ist, und der monoklonale Antikörper gegen das Nukleocapsid NP ein monoklonaler Antikörper ist, der durch ein Hybridom mit der Hinterlegungsnummer FERM BP-2124 hergestellt wird, wobei der monoklonale Antikörper stark an den Kern einer Zelle bindet, die mit dem Varicella zoster-Virus infiziert ist, wobei beide Antikörper mit Fluoresceinisothiocyanat markiert sind.

**9.** Diagnosekit nach Anspruch 8, wobei das Molverhältnis von Fluoreceinisothiocyanat und monoklonalem Antikörper zwischen 2,5 und 3,5 liegt.

**10.** Diagnosekit nach Anspruch 8 oder 9, wobei Evans' Blue zu den markierten monoklonalen Antikörpern gegeben wird.

**11.** Diagnosekit nach irgendeinem der Ansprüche 8 bis 10 mit:
(a) einer Lösung, enthaltend die beiden monoklonalen Antikörper oder
(b) einem Lyophilisat, enthaltend die beiden monoklonalen Antikörper oder
(c) getrennten Lösungen eines jeden der monoklonalen Antikörper oder
(d) getrennten Lyophilisaten eines jeden der monoklonalen Antikörper.

## Revendications

**1.** Procédé pour diagnostiquer la maladie infectieuse provoquée par le virus zostérien de la varicelle, comprenant la mise en contact d'un anticorps monoclone pour la glycoprotéine GP du virus zostérien de la varicelle et un anticorps monoclone pour la nucléocapside NP du virus zostérien de la varicelle avec un échantillon comprenant des cellules et la détection d'une liaison spécifique desdits deux anticorps avec des cellules infectées par ledit virus, caractérisé en ce que l'anticorps monoclone pour la glycoprotéine GP est un anticorps monoclone produit par un hybridome ayant le numéro de dépôt FERM BP-2123, anticorps monoclone qui se lie fortement au cytoplasme d'une cellule infectée par le virus zostérien de la varicelle et que l'anticorps monoclone pour la nucléocapside NP est un anticorps monoclone produit par un hybridome ayant le numéro de dépôt FERM BP-2124, anticorps monoclone qui se lie fortement au noyau d'une cellule infectée par le virus zostérien de la varicelle, dans lequel les deux anticorps sont marqués par de l'isothiocyanate de fluorescéine.

**2.** Procédé suivant la revendication 1, dans lequel le rapport molaire entre l'isothiocyanate de fluorescéine et l'anticorps monoclone est compris entre 2,5 et 3,5.

**3.** Procédé suivant la revendication 1 ou la revendication 2, dans lequel lesdits anticorps monoclones marqués sont utilisés en combinaison avec du Bleu d'Evans.

**4.** Agent de diagnostic pour la maladie infectieuse provoquée par le virus zostérien de la varicelle, comprenant un anticorps monoclone pour la glycoprotéine GP du virus zostérien de la varicelle et un anticorps monoclone pour la nucléocapside NP du virus zostérien de la varicelle, caractérisé en ce que l'anticorps monoclone pour la glycoprotéine GP est un anticorps monoclone produit par un hybridome ayant le numéro de dépôt FERM BP-2123, anticorps monoclone qui se lie fortement au cytoplasme d'une cellule infectée par le virus zostérien de la varicelle et que l'anticorps monoclone pour la nucléocapside NP est un anticorps monoclone produit par l'hybridome ayant le numéro de dépôt FERM BP-2124, anticorps monoclone qui se lie fortement au noyau d'une cellule infectée par le virus zostérien de la varicelle, dans lequel les deux anticorps sont marqués par de l'isothiocyanate de fluorescéine.

**5.** Agent de diagnostic suivant la revendication 4, dans lequel les deux anticorps monoclones sont mélangés l'un avec l'autre.

**6.** Agent de diagnostic suivant la revendication 4 ou la revendication 5, dans lequel le rapport molaire entre l'isothiocyanate de fluorescéine et l'anticorps monoclone est compris entre 2,5 et 3,5.

**7.** Agent de diagnostic suivant l'une ou l'autre des revendications 4, 5 ou 6, dans lequel du Bleu d'Evans est ajouté aux anticorps monoclones marqués.

**8.** Kit de diagnostic pour la maladie infectieuse provoquée par le virus zostérien de la varicelle, comprenant un anticorps monoclone pour la glycoprotéine GP du virus zostérien de la varicelle et un anticorps monoclone pour la nucléocapside NP du virus zostérien de la varicelle, caractérisé en ce que l'anticorps monoclone pour la glycoprotéine GP est un anticorps monoclone produit par un hybridome ayant le numéro de dépôt FERM BP-2123, anticorps monoclone qui se lie fortement au cytoplasme d'une cellule infectée par le virus zostérien de la varicelle et que l'anticorps monoclone pour la nucléocapside NP est un anticorps monoclone produit par l'hybridome ayant le numéro de dépôt FERM BP-2124, anticorps monoclone qui se lie fortement au noyau d'une cellule infectée par le virus zostérien de la varicelle, dans lequel les deux anticorps sont marqués par de l'isothiocyanate de fluorescéine.

9. Kit de diagnostic suivant la revendication 8, dans lequel le rapport molaire entre l'isothiocyanate de fluorescéine et l'anticorps monoclone est compris entre 2,5 et 3,5.

10. Kit de diagnostic suivant la revendication 8 ou la revendication 9, dans lequel du Bleu d'Evans est ajouté aux anticorps monoclones marqués.

11. Kit de diagnostic suivant l'une ou l'autre des revendications 8 à 10, comprenant:
    (a) une solution contenant les deux anticorps monoclones, ou
    (b) un lyophylisat contenant les deux anticorps monoclones, ou
    (c) des-solutions séparées de chacun des anticorps monoclones, ou
    (d) des lyophylisats séparés de chacun des anticorps monoclones.

*Fig. 1*

*Fig. 2.*